(19) Europäisches Patentamt
European Patent Office
Office européen des brevets

(11) Veröffentlichungsnummer: **0 037 971**
**B1**

(12) # EUROPÄISCHE PATENTSCHRIFT

(45) Veröffentlichungstag der Patentschrift: **21.12.83**

(51) Int. Cl.³: **C 07 C 129/14,** C 07 D 295/20, A 01 N 47/44

(21) Anmeldenummer: **81102484.3**

(22) Anmeldetag: **02.04.81**

(54) **Trisubstituierte Cyanguanidine, Verfahren zu ihrer Herstellung sowie ihre Verwendung als Fungizide.**

(30) Priorität: **15.04.80 DE 3014353**

(43) Veröffentlichungstag der Anmeldung: **21.10.81 Patentblatt 81/42**

(45) Bekanntmachung des Hinweises auf die Patenterteilung: **21.12.83 Patentblatt 83/51**

(84) Benannte Vertragsstaaten: **BE CH DE FR GB IT LI NL**

(56) Entgegenhaltungen:
**DE - A - 2 318 986**
**DE - A - 2 855 836**
**FR - A - 2 425 429**
**US - A - 4 138 561**

**CHEMICAL ABSTRACTS, Band 94, Nr. 7, 16. Februar 1981, Seite 591, Nr. 47428b Columbus, Ohio, U.S.A. E.J. KUPCHIK et al.: "Synthesis of N,N,N'-trisubstituted N"-cyano-guanidines and N-aryl-N'-(triorganostannyl)-N'N"-dicyanoguanidines"**

(73) Patentinhaber: **BAYER AG, Zentralbereich Patente, Marken und Lizenzen, D-5090 Leverkusen 1, Bayerwerk (DE)**

(72) Erfinder: **Niemers, Ekkehard, Dr., In den Birken 51a, D-5600 Wuppertal 1 (DE)**
Erfinder: **Lunkenheimer, Winfried, Dr., Bismarckstrasse 29, D-5600 Wuppertal 1 (DE)**
Erfinder: **Brandes, Wilhelm, Dr., Eichendorffstrasse 3, D-5653 Leichlingen (DE)**

**EP 0 037 971 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Trisubstituierte Cyanguanidine, Verfahren zu ihrer Herstellung sowie ihre Verwendung als Fungizide

Die vorliegende Erfindung betrifft trisubstituierte Cyanguanidine, mehrere Verfahren zu ihrer Herstellung sowie ihre Verwendung als Fungizide.

Es ist bereits bekanntgeworden, daß N-Chloracetyl-N-(2,6-dialkylphenyl)-alaninester, wie beispielsweise N-Chloracetyl-N-(2-ethyl-6-methyl-phenyl)-alaninethylester, mit gutem Erfolg zur Bekämpfung von pilzlichen Pflanzenkrankheiten eingesetzt werden können (vergleiche DE-OS 2 350 944). Deren Wirkung ist jedoch, insbesondere bei niedrigen Aufwandmengen und -konzentrationen, insbesondere auch bei der Bekämpfung von Phytophthora-Arten, nicht immer befriedigend.

Weiterhin sind trisubstituierte Cyanguanidine, wie z. B. N,N-Dimethyl-N'-methyl-N'-cyanoguanidin bekannt. Ihre Verwendung als Zwischenprodukte zur Herstellung von pharmakologisch wirksamen Verbindungen ist beschrieben, über eine Verwendung als Schädlingsbekämpfungsmittel ist nichts bekannt (vgl. FR-A-2 425 429).

Es wurden trisubstituierte Cyanguanidine der allgemeinen Formel

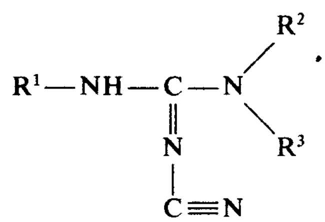

(I)

in welcher

- $R^1$ für gegebenenfalls substituiertes Phenyl und Phenylalkyl, Alkyl mit mehr als einen Kohlenstoffatom, substituiertes Alkyl, Alkenyl, Alkinyl oder gegebenenfalls substituiertes Cycloalkyl steht,
- $R^2$ für gegebenenfalls substituiertes Alkyl, Alkenyl oder Alkinyl steht,
- $R^3$ für gegebenenfalls substituiertes Alkyl, Alkenyl, Alkinyl, gegebenenfalls substituiertes Cycloalkyl oder gegebenenfalls substituiertes Phenyl und Phenylalkyl steht, sowie
- $R^2$ und $R^3$ gemeinsam mit dem N-Atom, an das sie gebunden sind, für einen gegebenenfalls substituierten Heterocyclus stehen; der gegebenenfalls ein O-Atom als weiteres Heteroatom enthält,

sowie deren physiologisch verträglichen Säureadditions-Salze gefunden.

Die erfindungsgemäßen Verbindungen der Formel (I) können in folgenden beiden tautomeren Formen vorliegen:

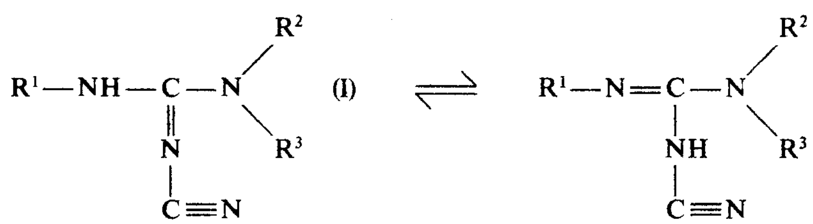

(I) (Ia)

Weiterhin wurde gefunden, daß man die trisubstituierten Cyanguanidine der Formel (I) erhält, wenn man

a) Thioharnstoffe der Formel

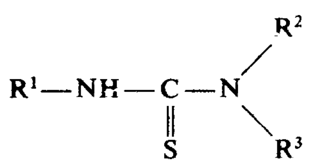

(II)

in welcher
$R^1$, $R^2$ und $R^3$ die oben angegebene Bedeutung haben,

mit einem Schwermetallsalz des Cyanamids in Gegenwart eines Verdünnungsmittels und gegebenenfalls in Gegenwart eines Phasentransferkatalysators umsetzt, oder

b) Amidine der Formel

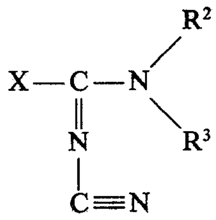

(III)

in welcher
$R^2$ und $R^3$ die oben angegebene Bedeutung haben und
X für Halogen, Alkoxy oder Alkylthio steht

mit Aminen der Formel

$$R^1-NH_2 \quad (IV)$$

in welcher
$R^1$ die oben angegebene Bedeutung hat,

in Gegenwart eines Verdünnungsmittels umsetzt, oder

c) Amidine der Formel

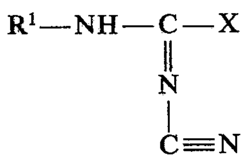

(V)

in welcher

$R^1$ die oben angegebene Bedeutung hat und
X für Halogen, Alkoxy und Alkylthio steht

mit Aminen der Formel

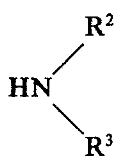

(VI)

in welcher
$R^2$ und $R^3$ die oben angegebene Bedeutung haben

in Gegenwart eines Verdünnungsmittels umsetzt.

Die neuen trisubstituierten Cyanguanidine weisen starke fungizide Eigenschaften auf. Dabei zeigen überraschenderweise die erfindungsgemäßen Verbindungen eine erheblich höhere Wirkung als der aus dem Stand der Technik bekannte N-Chlor-acetyl-N-(2-ethyl-6-methyl-phenyl)-alanin-ethylester. Die erfindungsgemäßen Stoffe stellen somit eine Bereicherung der Technik dar.

Die erfindungsgemäßen trisubstituierten Cyanguanidine sind durch die Formel (I) allgemein definiert. In dieser Formel steht $R^1$ vorzugsweise für gegebenenfalls einfach oder mehrfach, gleich oder verschieden substituiertes Phenyl, wobei als Substituenten vorzugsweise in Frage kommen: Halogen, Alkyl mit 1 bis 4 Kohlenstoffatomen, Alkenyl mit 2 bis 3 Kohlenstoffatomen, Alkoxy und Alkylthio mit jeweils 1 bis 4 Kohlenstoffatomen, Halogenalkyl mit 1 bis 2 Kohlenstoff- und 1 bis 5 gleichen oder verschiedenen Halogenatomen, wie insbesondere Fluor- und Chloratomen, Nitro, Cyano, Amino, Alkyl- und Dialkylamino mit 1 bis 4 Kohlenstoffatomen in jedem Alkylteil, Hydroxy, Alkylcarbonyl und Alkylsulfonyl mit jeweils 1 bis 4 Kohlenstoffatomen im Alkylteil, Carbamoyl, Aminosulfonyl, gegebenenfalls durch Halogen oder Alkyl mit 1 bis 2 Kohlenstoffatomen substituiertes Phenyl, Phenoxy oder Benzyloxy, sowie die Gruppierungen $-NR^4-CO-R^5$ und $-(CH_2)_n-NR^6R^7$, wobei $R^4$, $R^6$, $R^7$ vorzugsweise für Wasserstoff und Alkyl mit 1 bis 4 Kohlenstoffatomen stehen, $R^5$ vorzugsweise für Alkyl mit 1 bis 4 Kohlenstoffatomen steht und der Index n vorzugsweise für dieZahlen 0, 1, 2, 3 und 4 steht. $R^1$ steht außerdem vorzugsweise für gegebenenfalls im Phenylteil substituiertes

Phenylalkyl mit 1 oder 2 Kohlenstoffatomen im Alkylteil, wobei als Substituenten vorzugsweise in Frage kommen: Halogen, Alkyl mit 1 bis 4 Kohlenstoffatomen, Alkoxy und Alkylthio mit jeweils 1 bis 4 Kohlenstoffatomen, Halogenalkyl mit 1 bis 2 Kohlenstoff- und 1 bis 5 gleichen oder verschiedenen Halogenatomen, wie insbesondere Fluor- und Chloratomen, Nitro und Cyano. $R^1$ steht weiterhin vorzugsweise für geradkettiges oder verzweigtes Alkyl mit 2 bis 10 Kohlenstoffatomen sowie für substituiertes geradkettiges oder verzweigtes Alkyl mit 1 bis 10 Kohlenstoffatomen, wobei als Substituenten vorzugsweise in Frage kommen: Hydroxy, Cyano, Alkoxy mit 1 bis 4 Kohlenstoffatomen oder Cyclopropyl. $R^1$ steht weiterhin für Alkenyl und Alkinyl mit jeweils 2 bis 10 Kohlenstoffatomen sowie für gegebenenfalls durch Alkyl mit 1 bis 2 Kohlenstoffatomen substituiertes Cycloalkyl mit 3 bis 8 Kohlenstoffatomen. $R^2$ und $R^3$ stehen vorzugsweise für gegebenenfalls substituiertes, geradkettiges oder verzweigtes Alkyl mit 1 bis 4 Kohlenstoffatomen, wobei als Substituenten vorzugsweise in Frage kommen: Hydroxy, Cyano und Alkoxy mit 1 bis 4 Kohlenstoffatomen; sowie vorzugsweise für Alkenyl und Alkinyl mit jeweils 3 bis 5 Kohlenstoffatomen. $R^3$ steht außerdem noch vorzugsweise für gegebenenfalls durch Alkyl mit 1 bis 2 Kohlenstoffatomen substituiertes Cycloalkyl mit 5 bis 8 Kohlenstoffatomen, sowie für gegebenenfalls substituiertes Phenyl und Benzyl, wobei als Substituenten vorzugsweise die bei $R^1$ bereits vorzugsweise genannten Phenylsubstituenten in Frage kommen. $R^2$ und $R^3$ stehen außerdem gemeinsam mit dem N-Atom, an das sie gebunden sind, vorzugsweise für einen 5- bis 7gliedrigen Heterocyclus, der neben dem N-Atom gegebenenfalls noch ein O-Atom als weiteres Heteroatom enthält und gegebenenfalls substituiert sein kann durch Alkyl mit 1 bis 2 Kohlenstoffatomen oder durch einen ankondensierten Benzolring.

Besonders bevorzugt sind diejenigen trisubstituierten Cyanguanidine der Formel (I), in denen $R^1$ für gegebenenfalls einfach oder mehrfach, gleich oder verschieden substituiertes Phenyl steht, wobei als Substituenten vorzugsweise in Frage kommen: Fluor, Chlor, Brom, Jod, Methyl, Ethyl, Isopropanyl, Vinyl, Methoxy, Methylthio, Ethoxy, Isopropoxy, Trifluormethyl, Nitro, Cyano, Amino, Hydroxy, Dimethylamino, Acetyl, Methylsulfonyl, Carbamoyl, Aminosulfonyl, Phenyl, Chlorphenyl, Phenoxy, Chlorphenoxy, Benzyloxy, Chlorbenzyloxy, Methylaminomethyl, Dimethylaminomethyl, Methylaminoethyl, Dimethylaminoethyl, Acetylamino und Acetyl-methyl-amino; für gegebenenfalls einfach oder mehrfach gleich oder verschieden substituiertes Benzyl und Phenethyl steht, wobei als Substituenten vorzugsweise in Frage kommen: Fluor, Chlor, Methyl, Ethyl, Isopropyl, Methoxy, Methylthio, Trifluormethyl, Nitro und Cyano; für Cyclopropylmethyl, Cyclopropyl, Cyclobutyl, Cyclopentyl, gegebenenfalls durch Methyl und Ethyl substituiertes Cyclohexyl steht; $R^2$ für Methyl, Ethyl, n-Propyl, Isopropyl, Hydroxyethyl, Cyanethyl oder Allyl steht; $R^3$ für Methyl, Ethyl, n-Propyl, Isopropyl, Hydroxyethyl, Cyanethyl, Allyl, Cyclopentyl, gegebenenfalls durch Methyl oder Ethyl substituiertes Cyclohexyl, sowie für gegebenenfalls substituiertes Phenyl und Benzyl steht, wobei als Substituenten vorzugsweise in Frage kommen: Fluor, Chlor, Brom, Methyl, Ethyl, Methoxy, Nitro und Cyano; oder $R^2$ und $R^3$ gemeinsam mit dem N-Atom, an das sie gebunden sind, für gegebenenfalls durch Methyl oder einen ankondensierten Benzolring substituiertes Pyrrolidin, Piperidin oder Morpholin stehen.

Im einzelnen seien außer den bei den Herstellungsbeispielen genannten Verbindungen die folgenden Verbindungen der allgemeinen Formel (I) genannt:

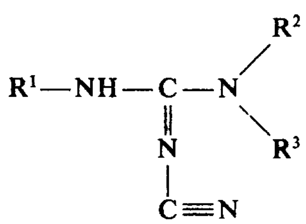

(I)

| $R^1$ | $R^2$ | $R^3$ |
|---|---|---|
| $2,6\text{-}Cl_2-C_6H_3$ | $-(CH_2)_4-$ | $-(CH_2)_4-$ |
| $2,6\text{-}Cl_2-C_6H_3$ | $CH_3$ | $CH_3$ |
| $2,6\text{-}(CH_3)_2-C_6H_3$ | $C_3H_7$ | $C_3H_7$ |
| $2,6\text{-}(CH_3)_2-C_6H_3$ | $-CH_2-CH=CH_2$ | $-CH_2-CH=CH_2$ |
| $2\text{-}CH_3-C_6H_4$ | $-(CH_2)_4-$ | $-(CH_2)_4-$ |
| $2\text{-}C_2H_5-C_6H_4$ | $-(CH_2)_4-$ | $-(CH_2)_4-$ |

Fortsetzung

| $R^1$ | $R^2$ | $R^3$ |
|---|---|---|
| $2\text{-i-}C_3H_7—C_6H_4$ | $—(CH_2)_4—$ | $—(CH_2)_4—$ |
| $2,3,6\text{-}(CH_3)_3—C_6H_4$ | $—(CH_2)_4—$ | $—(CH_2)_4—$ |
| 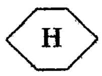 | $—(CH_2)_4—$ | $—(CH_2)_4—$ |
| 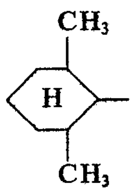 | $—(CH_2)_4—$ | $—(CH_2)_4—$ |
| 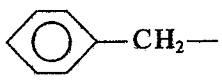 | $—(CH_2)_4—$ | $—(CH_2)_4—$ |
| 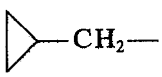 | $—(CH_2)_4—$ | $—(CH_2)_4—$ |
| 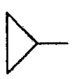 | $—(CH_2)_4—$ | $—(CH_2)_4—$ |
| 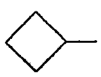 | $—(CH_2)_4—$ | $—(CH_2)_4—$ |
| 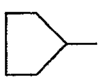 | $—(CH_2)_4—$ | $—(CH_2)_4—$ |
| 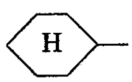 | $—(CH_2)_4—$ | $—(CH_2)_4—$ |
| 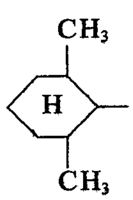 | $—(CH_2)_4—$ | $—(CH_2)_4—$ |
| 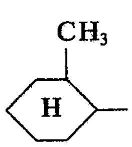 | $—(CH_2)_4—$ | $—(CH_2)_4—$ |
| 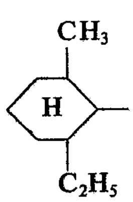 | $—(CH_2)_4—$ | $—(CH_2)_4—$ |

Fortsetzung

| $R^1$ | $R^2$ | $R^3$ |
|---|---|---|
| 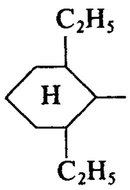 | $-(CH_2)_4-$ | $-(CH_2)_4-$ |
| 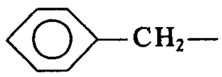 | $-(CH_2)_4-$ | $-(CH_2)_4-$ |
| 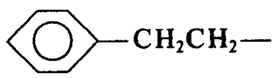 | $-(CH_2)_4-$ | $-(CH_2)_4-$ |
| 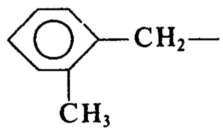 | $-(CH_2)_4-$ | $-(CH_2)_4-$ |
| 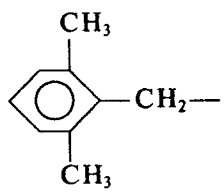 | $-(CH_2)_4-$ | $-(CH_2)_4-$ |
| 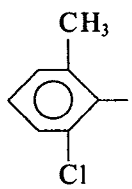 | $-(CH_2)_4-$ | $-(CH_2)_4-$ |
| 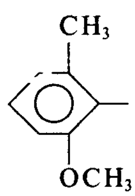 | $-(CH_2)_4-$ | $-(CH_2)_4-$ |
| 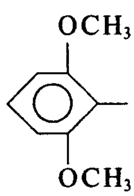 | $-(CH_2)_4-$ | $-(CH_2)_4-$ |
| 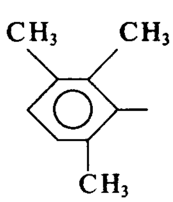 | $-(CH_2)_4-$ | $-(CH_2)_4-$ |
| 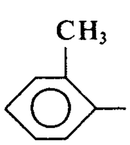 | $-(CH_2)_4-$ | $-(CH_2)_4-$ |

Fortsetzung

| $R^1$ | $R^2$ | $R^3$ |
|---|---|---|
| 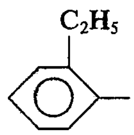 | $-(CH_2)_4-$ | $-(CH_2)_4-$ |
| 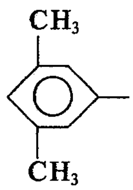 | $-(CH_2)_4-$ | $-(CH_2)_4-$ |
| 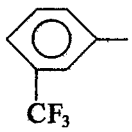 | $-(CH_2)_4-$ | $-(CH_2)_4-$ |
| 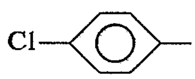 | $-(CH_2)_4-$ | $-(CH_2)_4-$ |
| 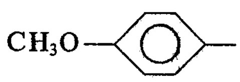 | $-(CH_2)_4-$ | $-(CH_2)_4-$ |
| 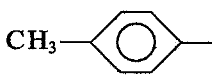 | $-(CH_2)_4-$ | $-(CH_2)_4-$ |
| 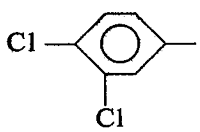 | $-(CH_2)_4-$ | $-(CH_2)_4-$ |
| 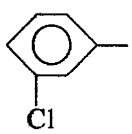 | $-(CH_2)_4-$ | $-(CH_2)_4-$ |
| 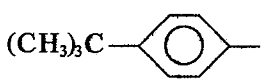 | $-(CH_2)_4-$ | $-(CH_2)_4-$ |
| 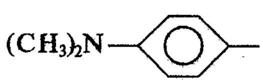 | $-(CH_2)_4-$ | $-(CH_2)_4-$ |
| 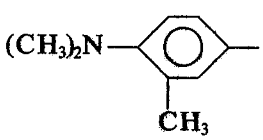 | $-(CH_2)_4-$ | $-(CH_2)_4-$ |
| 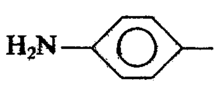 | $-(CH_2)_4-$ | $-(CH_2)_4-$ |
| 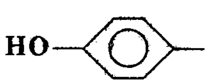 | $-(CH_2)_4-$ | $-(CH_2)_4-$ |
| 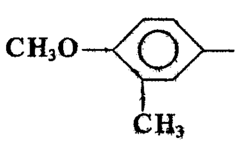 | $-(CH_2)_4-$ | $-(CH_2)_4-$ |

Fortsetzung

| $R^1$ | $R^2$ | $R^3$ |
|---|---|---|
| 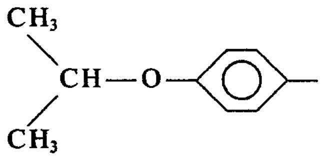 | $-(CH_2)_4-$ | $-(CH_2)_4-$ |
| 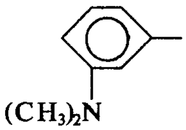 | $-(CH_2)_4-$ | $-(CH_2)_4-$ |
| 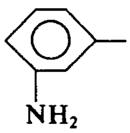 | $-(CH_2)_4-$ | $-(CH_2)_4-$ |
| 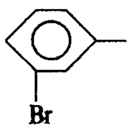 | $-(CH_2)_4-$ | $-(CH_2)_4-$ |
| 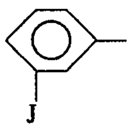 | $-(CH_2)_4-$ | $-(CH_2)_4-$ |
| 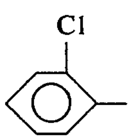 | $-(CH_2)_4-$ | $-(CH_2)_4-$ |
| 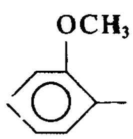 | $-(CH_2)_4-$ | $-(CH_2)_4-$ |
| 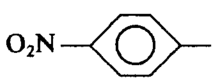 | $-(CH_2)_4-$ | $-(CH_2)_4-$ |
| 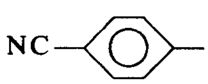 | $-(CH_2)_4-$ | $-(CH_2)_4-$ |
| 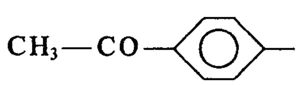 | $-(CH_2)_4-$ | $-(CH_2)_4-$ |
| 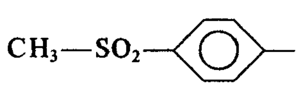 | $-(CH_2)_4-$ | $-(CH_2)_4-$ |
| 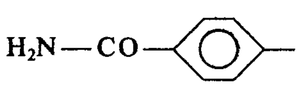 | $-(CH_2)_4-$ | $-(CH_2)_4-$ |
| 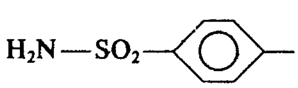 | $-(CH_2)_4-$ | $-(CH_2)_4-$ |
| 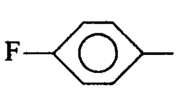 | $-(CH_2)_4-$ | $-(CH_2)_4-$ |

Fortsetzung

| $R^1$ | $R^2$ | $R^3$ |
|---|---|---|
| 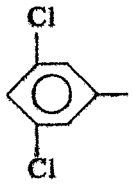 | $-(CH_2)_4-$ | $-(CH_2)_4-$ |
| 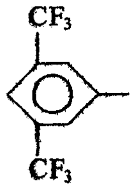 | $-(CH_2)_4-$ | $-(CH_2)_4-$ |
| 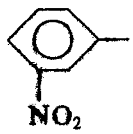 | $-(CH_2)_4-$ | $-(CH_2)_4-$ |
| 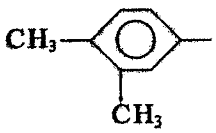 | $-(CH_2)_4-$ | $-(CH_2)_4-$ |
| 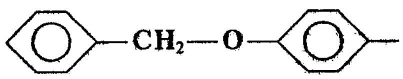 | $-(CH_2)_4-$ | $-(CH_2)_4-$ |
| 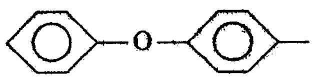 | $-(CH_2)_4-$ | $-(CH_2)_4-$ |
| 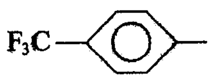 | $-(CH_2)_4-$ | $-(CH_2)_4-$ |
| 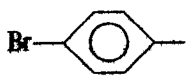 | $-(CH_2)_4-$ | $-(CH_2)_4-$ |
| 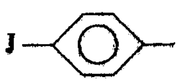 | $-(CH_2)_4-$ | $-(CH_2)_4-$ |
| 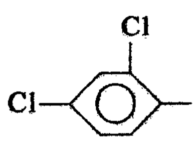 | $-(CH_2)_4-$ | $-(CH_2)_4-$ |
| 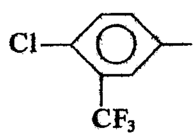 | $-(CH_2)_4-$ | $-(CH_2)_4-$ |
| 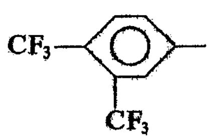 | $-(CH_2)_4-$ | $-(CH_2)_4-$ |
| 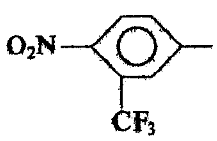 | $-(CH_2)_4-$ | $-(CH_2)_4-$ |

Fortsetzung

| $R^1$ | $R^2$ | $R^3$ |
|---|---|---|
| 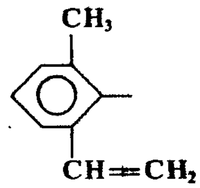 | $-(CH_2)_4-$ | $-(CH_2)_4-$ |
| 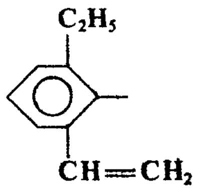 | $-(CH_2)_4-$ | $-(CH_2)_4-$ |
| 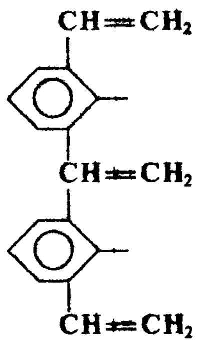 | $-(CH_2)_4-$ | $-(CH_2)_4-$ |
| | $-(CH_2)_4-$ | $-(CH_2)_4-$ |
| 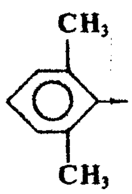 | $-CH(CH_3)_2$ | $-CH(CH_3)_2$ |
| 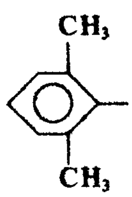 | $-CH_2-CH=CH_2$ | $-CH_2-CH=CH_2$ |
| 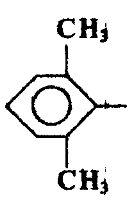 | $-CH_2CH_2OH$ | $CH_3$ |
| 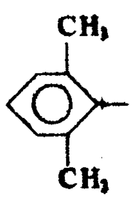 | $-CH_2CH_2OH$ | $-CH_2CH_2OH$ |

Fortsetzung

| $R^1$ | $R^2$ | $R^3$ |
|---|---|---|
| 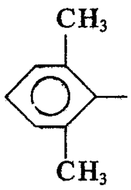 | $-CH_2CH_2OH$ | $CH_3$ |
| 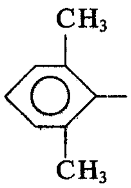 | $CH_3$ | 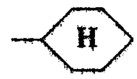 |
| 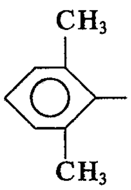 | $CH_3$ | 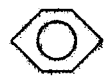 |
| 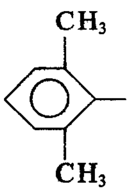 | $CH_3$ | $-CH_2-$ 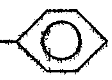 |
| 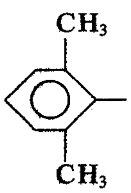 | 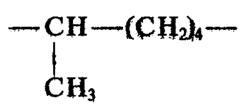 | 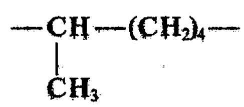 |
| 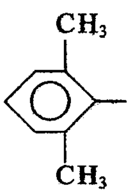 | 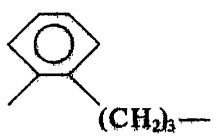 | 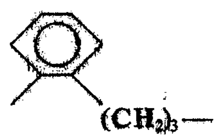 |
| 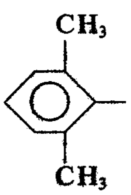 | 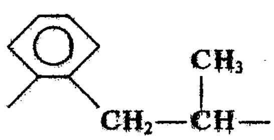 | 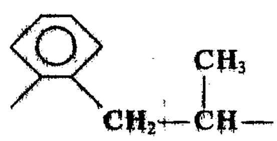 |
| 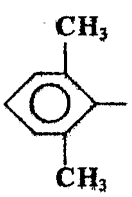 | $-(CH_2)_6-$ | $-(CH_2)_6-$ |

**Verwendet man beispielsweise N'-(2,6-Dimethylphenyl)-N,N-tetramethylen-thioharnstoff und Blei-cyanamid als Ausgangsstoffe, so kann der Reaktionsablauf durch das folgende Formelschema wiedergegeben werden (Verfahren a):**

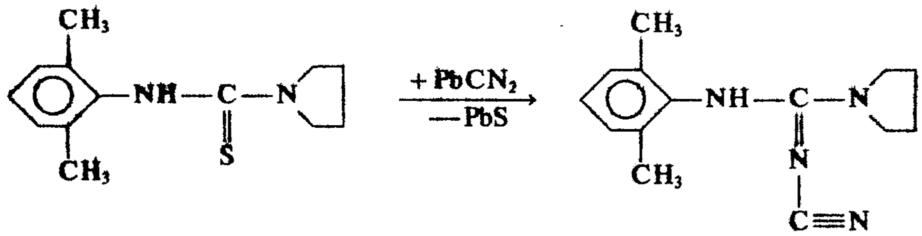


**Verwendet man beispielsweise N-Cyano-N',N'-dimethyl-S-methyl-isothioharnstoff und Benzylamin als Ausgangsstoffe, so kann der Reaktionsablauf durch das folgende Formelschema wiedergegeben werden (Verfahren b):**

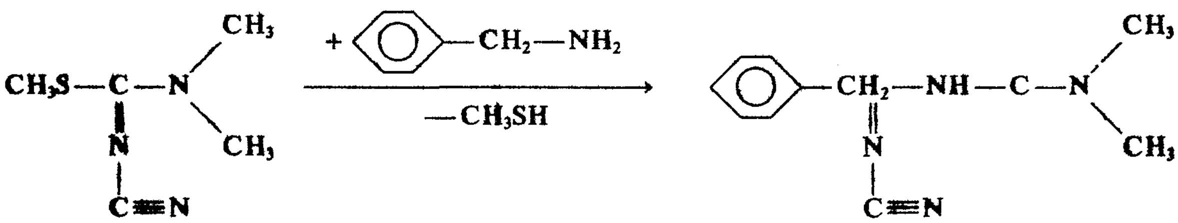


**Verwendet man beispielsweise N-tert.-Butyl-N'-cyan-S-methyl-isothioharnstoff und Pyrrolidin als Ausgangsstoffe, so kann der Reaktionsablauf durch das folgende Formelschema wiedergegeben werden (Verfahren c):**

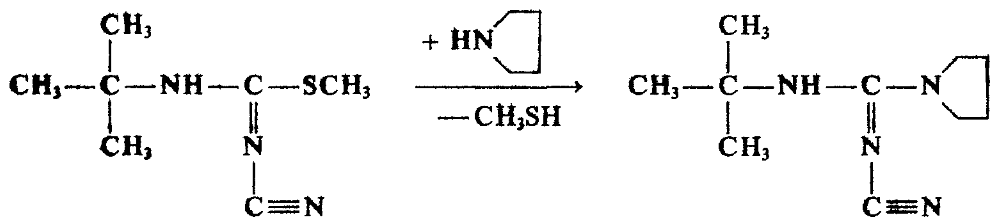


Die bei der Durchführung des erfindungsgemäßen Verfahrens (a) als Ausgangsstoffe benötigten Thioharnstoffe sind durch die Formel (II) allgemein definiert. In dieser Formel stehen $R^1$, $R^2$ und $R^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die Thioharnstoffe der Formel (II) sind allgemein bekannte Verbindungen der organischen Chemie.

Die außerdem für das erfindungsgemäße Verfahren (a) als Ausgangsstoffe benötigten Schwermetallsalze des Cyanamids sind ebenfalls bekannt. Als Schwermetallsalze werden vorzugsweise Blei-, Quecksilber- und Cadmiumsalze eingesetzt.

Die bei der Durchführung des erfindungsgemäßen Verfahrens (b) als Ausgangsstoffe benötigten Amidine sind durch die Formel (III) allgemein definiert. In dieser Formel steht X vorzugsweise für Chlor, Brom, Methoxy oder Methylmercapto; $R^2$ und $R^3$ stehen vorzugsweise fü diejenigen Reste, die bereits im Zusammenhang mit der Beschreibung der erfindungsgemäßen Stoffe der Formel (I) vorzugsweise für diese Substituenten genannt wurden.

Die Amidine der Formeln (III) und (V) sind bekannt; bzw. können sie in allgemein bekannter Art und Weise erhalten werden, indem man z. B. 1 Mol eines Cyanamidodithiokohlensäuredialkylesters in Gegenwart eines polaren Lösungsmittels, wie beispielsweise eines Alkohols, zwischen 0 und 150°C mit 1 Mol eines entsprechenden Amins unter Abspaltung von 1 Mol Alkylmercaptan umsetzt.

Als Verdünnungsmittel kommen für die erfindungsgemäße Umsetzung gemäß Verfahren (a) vorzugsweise inerte organische Lösungsmittel in Frage. Hierzu gehören vorzugsweise Alkohole, wie Methanol und Ethanol, aber auch mehrwertige Alkohole, wie Glykol, und deren veretherte Derivate; Ether, wie Tetrahydrofuran und Dioxan; Nitrile, wie Acetonitril; und Amide, wie Dimethylformamid und Dimethylacetamid.

Das erfindungsgemäße Verfahren (a) wird gegebenenfalls in Gegenwart eines Phasentransferkatalysators durchgeführt, wie beispielsweise Ammonium- oder Phosphoniumverbindungen, wie z. B. Triethyl-benzyl-ammoniumchlorid.

Die Reaktionstemperaturen können bei der Durchführung des erfindungsgemäßen Verfahrens (a) in einem größeren Bereich variiert werden. Im allgemeinen arbeitet man zwischen 50 und 200°C, vorzugsweise zwischen 70 und 160°C.

Bei der Durchführung des erfindungsgemäßen Verfahrens (a) setzt man vorzugsweise auf 1 Mol Thioharnstoff der Formel (II) 2 Mol an Schwermetallsalz des Cyanamids und gegebenenfalls 0,01 bis 0,1 Mol des Phasentransferkatalysators ein. Die Isolierung der Verbindungen der Formel (I) erfolgt in üblicher und bekannter Weise.

In einer besonderen Ausführungsform des erfindungsgemäßen Verfahrens (a) wird zwischendurch sich bildendes Schwermetallsulfid einschließlich noch nicht umgesetztem Cyanamid abfiltriert und frisches Cyanamid sowie gegebenenfalls auch Phasentransferkatalysator wieder zugegeben.

Als Verdünnungsmittel kommen für die erfindungsgemäße Umsetzung gemäß Verfahren (b) und (c) vorzugsweise polare organische Lösungsmittel in Frage. Hierzu gehören vorzugsweise Alkohole, wie Methanol und Ethanol; Ether, wie Diethylether, Dioxan und Tetrahydrofuran; Nitrile, wie Acetonitril; sowie Amide, wie Dimethylformamid.

Die Reaktionstemperaturen können bei der Durchführung der erfindungsgemäßen Verfahren (b) und (c) in einem größeren Bereich variiert werden. Im allgemeinen arbeitet man zwischen 0 und 150°C, vorzugsweise zwischen 20 und 150°C.

Bei der Durchführung der erfindungsgemäßen Verfahren (b) und (c) arbeitet man vorzugsweise in molaren Mengen. Die Isolierung der Verbindungen der Formel (I) erfolgt in allgemein üblicher und bekannter Weise.

Die erfindungsgemäßen Stoffe der Formel (I) können außer nach den angegebenen Verfahren (a), (b) und (c) auch nach anderen, allgemein bekannten Verfahrensvarianten erhalten werden, wie z. B. durch Umsetzung von

Amidinen der Formel

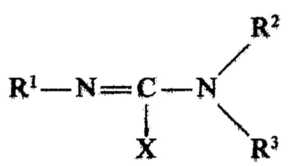

(VII)

in welcher
$R^1$, $R^2$, $R^3$ und X die oben angegebene Bedeutung haben,

mit Cyanamid in allgemein üblicher und bekannter Weise, oder durch Umsetzung von

Guanidinen der Formel

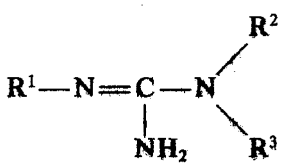

(VIII)

in welcher
$R^1$, $R^2$ und $R^3$ die oben angegebene Bedeutung haben,

mit Brom(Chlor)cyan in allgemein üblicher und bekannter Weise.

Zur Herstellung von physiologisch verträglichen Säureadditionssalzen der Verbindungen der Formel (I) kommen vorzugsweise folgende Säuren in Frage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z. B. gegen den Erreger der Kraut- und Braunfäule der Tomate und Kartoffel (Phytophthora infestans) eingesetzt werden. Besonders hervorzuheben ist, daß die erfindungsgemäßen Wirkstoffe nicht nur eine protektive, sondern auch eine systemische Wirkung besitzen. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägersoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemmäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

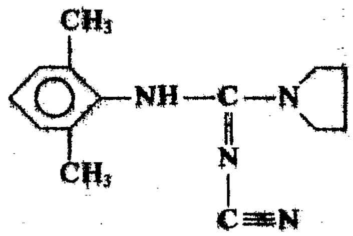

(Verfahren a)

Zu einer Lösung von 234,4 g (1 Mol) N'-(2,6-Dimethylphenyl)-N,N-tetramethylen-thioharnstoff in 1000 ml Dimethylformamid werden 247,2 g (1 Mol) Bleicyanamid gegeben. Das Reaktionsgemisch wird 48 Stunden bei 135°C gerührt. Dabei werden nach 7 und 17 Stunden jeweils weitere 123,6 g (0,5 Mol) Bleicyanamid zugegeben. Das Reaktionsgemisch wird heiß filtriert, das Filtrat eingeengt und der Rückstand aus Ethanol umkristallisiert. Man erhält 148,6 g (61,3% der Theorie) N''-Cyano-N'-(2,6-dimethylphenyl)-N,N-tetramethylen-guanidin vom Schmelzpunkt 192–194°C.

In analoger Weise und gemäß den Verfahren (a), (b) und (c) werden die folgenden Verbindungen der allgemeinen Formel (I)

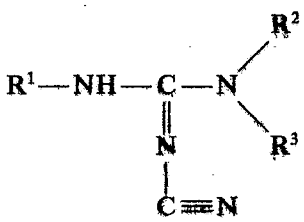

(I)

erhalten:

| Bsp. Nr. | $R^1$ | $R^2$ | $R^3$ | Schmelzpunkt (°C) |
|---|---|---|---|---|
| 2 | $2,6-(CH_3)_2-C_6H_3$ | $CH_3$ | $CH_3$ | 246–247 (Zers.) |
| 3 | $4-Cl, 2-CH_3-C_6H_3$ | $CH_3$ | $CH_3$ | 205–207 |
| 4 | $2-Cl, 6-CH_3-C_6H_3$ | $CH_3$ | $CH_3$ | 243–244 |
| 5 | $2,6-(i-C_3H_7)_2C_6H_3$ | $CH_3$ | $CH_3$ | 218–219 |
| 6 | $2,4,6-(CH_3)_3-C_6H_2$ | $CH_3$ | $CH_3$ | 230–232 |
| 7 | $2,6-(CH_3)_2-C_6H_3$ | $-(CH_2)_5-$ | $-(CH_2)_5-$ | 196–198 |
| 8 | $2,6-(CH_3)_2-C_6H_3$ | $C_2H_5$ | $C_2H_5$ | 169–175 |
| 9 | $2-Cl, 6-CH_3-C_6H_3$ | $-(CH_2)_5-$ | $-(CH_2)_5-$ | 165–168 |
| 10 | $2,4,6-(CH_3)_3C_6H_2$ | $-(CH_2)_4-$ | $-(CH_2)_4-$ | 161 |
| 11 | $2,6-(CH_3)_2-C_6H_3$ | $-(CH_2)_2-O-(CH_2)_2-$ | $-(CH_2)_2-O-(CH_2)_2-$ | 192–195 |
| 12 | $C_6H_5$ | $-(CH_2)_4-$ | $-(CH_2)_4-$ | 166–168 |
| 13 | $C_6H_5$ | $CH_3$ | $CH_3$ | 162–164 |
| 14 | $2,6-Cl_2-C_6H_3$ | $-(CH_2)_2-O-(CH_2)_2-$ | $-(CH_2)_2-O-(CH_2)_2-$ | 212 |
| 15 | $2,6-(C_2H_5)_2-C_6H_3$ | $-(CH_2)_4-$ | $-(CH_2)_4-$ | 176–178 |
| 16 | $2-C_2H_5, 6-CH_3-C_6H_3$ | $-(CH_2)_4-$ | $-(CH_2)_4-$ | 165–168 (Zers.) (× HCl) |
| 17 | $2,6-(CH_3)_2-C_6H_3$ | $-(CH_2)_6-$ | $-(CH_2)_6-$ | 176–180 |
| 18 | $2-Cl, 6-CH_3-C_6H_3$ | $-(CH_2)_4-$ | $-(CH_2)_4-$ | 190–192 |
| 19 | $2,6-(CH_3)_2-C_6H_3$ | $C_3H_7$ | $C_3H_7$ | 89–92 |
| 20 | $2-CH_3, 6-OCH_3-C_6H_3$ | $-(CH_2)_4-$ | $-(CH_2)_4-$ | 153–154 |
| 21 | $2-CH_3-C_6H_4$ | $-(CH_2)_4-$ | $-(CH_2)_4-$ | 155–157 |
| 22 | $2-CF_3-C_6H_4$ | $-(CH_2)_4-$ | $-(CH_2)_4-$ | 143–144 |
| 23 | $2,5-(CH_3)_2-C_6H_3$ | $-(CH_2)_4$ | $-(CH_2)_4$ | 177–178 |
| 24 | $2,3-(CH_3)_2-C_6H_3$ | $-(CH_2)_4-$ | $-(CH_2)_4-$ | 186–190 |
| 25 | $2,4-(CH_3)_2-C_6H_3$ | $-(CH_2)_4-$ | $-(CH_2)_4-$ | 157–159 |
| 26 | $3,4-(CH_3)_2-C_6H_3$ | $-(CH_2)_4-$ | $-(CH_2)_4-$ | 172–175 |
| 27 | $2-OCH_3, 4-CH_3-C_6H_3$ | $-(CH_2)_4$ | $-(CH_2)_4$ | 178–181 |

## Anwendungsbeispiele

In den nachfolgenden Beispielen wird die nachstehend angegebene Verbindung als Vergleichssubstanz eingesetzt:

(A) = 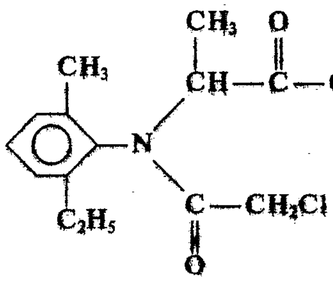

N-Chloracetyl-N-(2-ethyl-6-methyl-phenyl)-alanin-ethylester

### Beispiel A

Phytophthora-Test (Tomaten)/Protektiv

Lösungsmittel: 4,7 Gewichtsteile Aceton
Emulgator: 0,3 Gewichtsteile Alkyl-aryl-polyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20° C und einer relativen Luftfeuchtigkeit von 70% im Gewächshaus. Anschließend werden die Tomatenpflanzen mit einer wäßrigen Sporensuspension von Phytophthora infestans inokuliert. Die Pflanzen werden in eine Feuchtkammer mit einer 100%igen Luftfeuchtigkeit und einer Temperatur von 18 bis 20° C gebracht.

Nach 5 Tagen wird der Befall der Tomatenpflanzen bestimmt. Die erhaltenen Boniturwerte werden auf Prozent Befall umgerechnet. 0% bedeutet keinen Befall, 100% bedeutet, daß die Pflanzen vollständig befallen sind.

In diesem Test zeigen z. B. folgende Verbindungen eine sehr gute Wirkung, die derjenigen der aus dem Stand der Technik bekannten Verbindung (A) deutlich überlegen ist:

Verbindungen gemäß Herstellungsbeispielen 1, 2 und 8.

### Beispiel B

Phytophthora-Test (Tomaten)/Systemisch

Lösungsmittel: 4,7 Gewichtsteile Aceton
Dispergiermittel: 0,3 Gewichtsteile Alkyl-aryl-polyglykolether
Wasser: 95,0 Gewichtsteile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Tomatenpflanzen mit 2 bis 4 Laubblättern werden mit 10 ml der Gießflüssigkeit in der angegebenen Wirkstoffkonzentration, bezogen auf 100 ml Erde, gegossen.

Die so behandelten Pflanzen werden nach der Behandlung mit einer wäßrigen Sporensuspension von Phytophthora infestans inokuliert. Die Pflanzen werden in eine Feuchtkammer mit einer Luftfeuchtigkeit von 100% und einer Temperatur von 18 bis 20° C gebracht. Nach 5 Tagen wird der Befall der Tomatenpflanzen bestimmt. Die so erhaltenen Boniturwerte werden auf Prozent Befall umgerechnet. 0% bedeutet keinen Befall, 100% bedeutet, daß die Pflanzen vollständig befallen sind.

In diesem Test zeigen z. B. folgende Verbindungen eine sehr gute Wirkung, die derjenigen der aus dem Stand der Technik bekannten Verbindung (A) deutlich überlegen ist:

Verbindungen gemäß Herstellungsbeispielen 1, 2, 3, 4, 8 und 9.

**Patentansprüche**

1. Trisubstituierte Cyanguanidine der Formel I

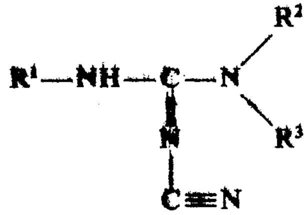

(I)

in welcher

$R^1$ für Phenyl steht, welches gegebenenfalls durch Halogen, Alkyl und Alkoxy mit jeweils bis zu 3 Kohlenstoffatomen, durch Vinyl, Methylthio, Nitro, Cyano, Amino, Hydroxy, Dimethylamino, Acetyl, Carbamoyl, Aminosulfonyl, Phenyl, Chlorphenyl, Phenoxy, Chlorphenoxy, Benzyloxy, Chlorbenzyloxy, Methylaminomethyl, Dimethylaminomethyl, Methylaminoethyl, Dimethylaminoethyl, Acetylamino und Acetyl-methyl-amino substituiert sein kann; für Benzyl und Phenylethyl steht, wobei die beiden genannten Reste substituiert sein können durch Fluor, Chlor, Alkyl mit bis zu 3 Kohlenstoffatomen, Methoxy, Methylthio, Nitro und Cyano; für Cyclopropylmethyl und für Cyclopropyl, Cyclobutyl, Cyclopentyl und Cyclohexyl steht, wobei die letztgenannte Gruppe durch Methyl und Ethyl substituiert sein kann;

$R^2$ für gegebenenfalls substituiertes Alkyl, Alkenyl oder Alkinyl steht,

$R^3$ für gegebenenfalls substituiertes Alkyl, Alkenyl, Alkinyl, gegebenenfalls substituiertes Cycloalkyl oder gegebenenfalls substituiertes Phenyl und Phenylalkyl steht, sowie

$R^2$ und $R^3$ gemeinsam mit dem N-Atom, an das sie gebunden sind, für einen gegebenenfalls substituierten Heterocyclus stehen, der gegebenenfalls ein O-Atom als weiteres Heteroatom enthält,

sowie deren physiologisch verträglichen Säureadditions-Salze.

2. Verfahren zur Herstellung von trisubstituierten Cyanguanidinen der Formel I, dadurch gekennzeichnet, daß man

a) Thioharnstoffe der Formel

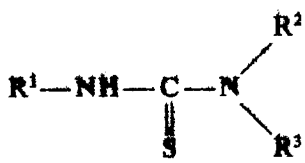

(II)

in welcher
$R^1$, $R^2$ und $R^3$ die in Anspruch 1 angegebene Bedeutung haben,

mit einem Schwermetallsalz des Cyanamids in Gegenwart eines Verdünnungsmittels und gegebenenfalls in Gegenwart eines Phasentransferkatalysators umsetzt, oder

b) Amidine der Formel

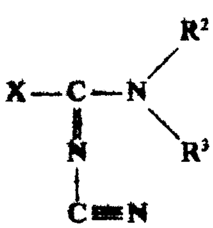

(III)

in welcher
$R^2$ und $R^3$ die in Anspruch 1 angegebene Bedeutung haben und
X für Halogen, Alkoxy oder Alkylthio steht

mit Aminen der Formel

$R^1-NH_2$ (IV)

in welcher
$R^1$ die in Anspruch 1 angegebene Bedeutung hat,

in Gegenwart eines Verdünnungsmittels umsetzt, oder

c) Amidine der Formel

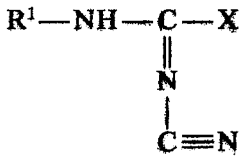

(V)

in welcher

$R^1$ die in Anspruch 1 angegebene Bedeutung hat und
X für Halogen, Alkoxy und Alkylthio steht

mit Aminen der Formel

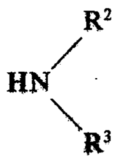

(VI)

in welcher
$R^2$ und $R^3$ die in Anspruch 1 angegebene Bedeutung haben

in Gegenwart eines Verdünnungsmittels umsetzt.

3. Fungizide Mittel, gekennzeichnet durch einen Gehalt an mindestens einem trisubstituierten Cyanguanidin der Formel I.

4. Verfahren zur Bekämpfung von Pilzen, dadurch gekennzeichnet, daß man trisubstituierte Cyanguanidine der Formel I auf Pilze oder ihren Lebensraum einwirken läßt.

5. Verwendung von trisubstituierten Cyanguanidinen der Formel I zur Bekämpfung von Pilzen.

6. Verfahren zur Herstellung von fungiziden Mitteln, dadurch gekennzeichnet, daß man trisubstituierte Cyanguanidine der Formel I mit Streckmitteln und/oder oberflächenaktiven Mitteln vermischt.

## Claims

1. Trisubstituted cyanoguanidines of the formula I

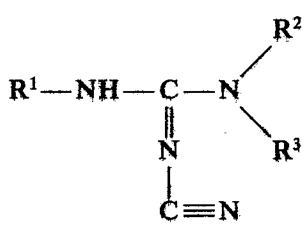

(I)

in which

$R^1$ represents phenyl, which can optionally be substituted by halogen, alkyl or alkoxy with in each case up to 3 carbon atoms, by vinyl, methylthio, nitro, cyano, amino, hydroxyl, dimethylamino, acetyl, carbamoyl, aminosulphonyl, phenyl, chlorophenyl, phenoxy, chlorophenoxy, benzyloxy, chlorobenzyloxy, methylaminomethyl, dimethylaminomethyl, methylaminoethyl, dimethylaminoethyl, acetylamino or acetyl-methyl-amino; benzyl or phenylethyl, it being possible for the two radicals mentioned to be substituted by fluorine, chlorine, alkyl with up to 3 carbon atoms, methoxy, methylthio, nitro or cyano; cyclopropylmethyl or cyclopropyl, cyclobutyl, cyclopentyl or cyclohexyl, it being possible for the group mentioned last to be substituted by methyl or ethyl;
$R^2$ represents optionally substituted alkyl, alkenyl or alkinyl and
$R^3$ represents optionally substituted alkyl, alkenyl, alkinyl, optionally substituted cycloalkyl or optionally substituted phenyl or phenylalkyl, or

$R^2$ and $R^3$, together with the N atom to which they are bonded, represent an optionally substituted heterocyclic radical, which optionally contains an O atom as a further hetero-atom,

and physiologically acceptable acid addition salts thereof.

2. Process for the preparation of trisubstituted cyanoguanidines of the formula I, characterised in that

a) thioureas of the formula

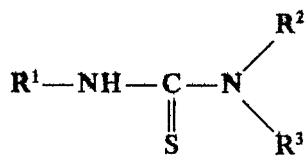

(II)

in which
$R^1$, $R^2$ and $R^3$ have the meaning given in Claim 1, are reacted with a heavy metal salt of cyanamide in the presence of a diluent and if appropriate in the presence of a phase transfer catalyst, or

b) amidines of the formula

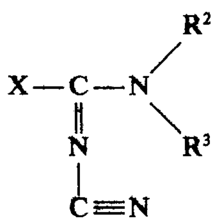

(III)

in which
$R^2$ and $R^3$ have the meaning given in Claim 1 and
X represents halogen, alkoxy or alkylthio,

are reacted with amines of the formula

$R^1—NH_2$ (IV)

in which
$R^1$ has the meaning given in Claim 1,

in the presence of a diluent, or

c) amidines of the formula

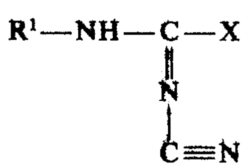

(V)

in which

$R^1$ has the meaning given in Claim 1 and
X represents halogen, alkoxy or alkylthio,

are reacted with amines of the formula

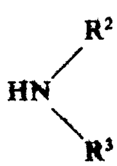

(VI)

in which
$R^2$ and $R^3$ have the meaning given in Claim 1,

in the presence of a diluent.

3. Fungicidal agents, characterised in that they contain at least one trisubstituted cyanoguanidine of the formula I.

4. Process for combating fungi, characterised in that trisubstituted cyanoguanidines of the formula I are allowed to act on fungi or their environment.

5. Use of trisubstituted cyanoguanidines of the formula I for combating fungi.

6. Process for the preparation of fungicidal agents, characterised in that trisubstituted cyanoguanidines of the formula I are mixed with extenders and/or surfaceactive agents.

**Revendications**

1. Cyanoguanidines trisubstituées de formule I

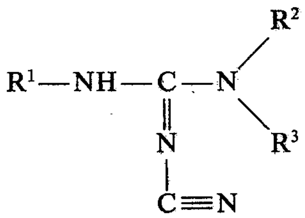

(I)

dans laquelle

$R^1$ est un groupe phényle, qui est éventuellement substitué par un halogène, un radical alkyle et un radical alkoxy ayant chacun jusqu'à 3 atomes de carbone, un radical vinyle, méthylthio, nitro, cyano, amino, hydroxy, diméthylamino, acétyle, carbamoyle, aminosulfonyle, phényle, chlorophényle, phénoxy, chlorophénoxy, benzyloxy, chlorobenzyloxy, méthylaminométhyle, diméthylaminométhyle, méthylaminoéthyle, diméthylaminoéthyle, acétylamino et acétylméthyl-amino; un reste benzyle, et un reste phényléthyle, les deux restes mentionnés pouvant être substitués par du fluor, du chlore, un radical alkyle ayant jusqu'à 3 atomes de carbone, méthoxy, méthylthio, nitro et cyano; un groupe cyclopropylméthyle et un groupe cyclopropyle, cyclobutyle, cyclopentyle et cyclohexyle, le groupe mentionné en dernier lieu pouvant être substitué par un radical méthyle et un radical éthyle;

$R^2$ est un groupe alkyle, alcényle ou alcynyle éventuellement substitué,

$R^3$ est un groupe alkyle, alcényle, alcynyle éventuellement substitué, cycloalkyle éventuellement substitué ou phényle et phénylalkyle éventuellement substitués, de même que

$R^2$ et $R^3$ forment conjointement avec l'atome d'azote auquel ils sont liés un hétérocycle éventuellement substitué qui comporte le cas échéant un atome d'oxygène comme autre hétéro-atome,

ainsi que leurs sels d'addition d'acides physiologiquement acceptables.

2. Procédé de production de cyanoguanidines trisubstitués de formule I, caractérisé en ce que

a) on fait réagir des thiourées de formule

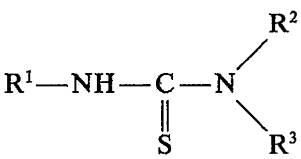

(II)

dans laquelle
$R^1$, $R^2$ et $R^3$ ont la définition indiquée dans la revendication 1,

avec un sel de métal lourd du cyanamide en présence d'un diluant et, le cas échéant, en présence d'un catalyseur de transfert de phase, ou

b) on fait réagir des amidines de formule

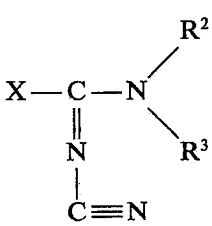

(III)

dans laquelle
$R^2$ et $R^3$ ont la définition indiquée dans la revendication 1 et
X est un halogène, un groupe alkoxy ou un groupe alkylthio,

avec des amines de formule

$$R^1—NH_2 \qquad (IV)$$

dans laquelle
$R^1$ a la définition indiquée dans la revendication 1,

en présence d'un diluant, ou

c) on fait réagir des amidine de formule

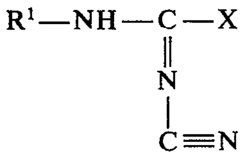

(V)

dans laquelle

$R^1$ a la définition indiquée dans la revendication 1 et
X est un halogène, un groupe alkoxy et un groupe alkylthio,

avec des amines de formule

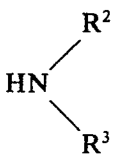

(VI)

dans laquelle
$R^2$ et $R^3$ ont la définition indiquée dans la revendication 1

en présence d'un diluant.

3. Compositions fongicides, caractérisées par une teneur en au moins une cyanoguanidine trisubstituée de formule I.

4. Procédé pour combattre des champignons, caractérisé en ce qu'on fait agir des cyanoguanidines trisubstituées de formule I sur des champignons ou sur leur milieu.

5. Utilisation de cyanoguanidines trisubstituées de formule I pour lutter contre les champignons.

6. Procédé de préparation de compositions fongicides, caractérisé en ce qu'on mélange des cyanoguanidines trisubstituées de formule I avec des diluants et/ou des agents tensio-actifs.